# EUROPÄISCHE PATENTSCHRIFT

(11) **EP 1 429 813 B1**
(45) Veröffentlichungstag und Bekanntmachung des Hinweises auf die Patenterteilung: **20.04.2005**
(21) Anmeldenummer: 02798736.1
(22) Anmeldetag: 18.09.2002
(51) Int. Cl.: A61L 9/03, A61L 9/02, A01M 31/00

(54) **VORRICHTUNG ZUM GESTEUERTEN FREISETZEN MEHRERER DUFT-/LOCK-STOFFE UND VERFAHREN ZUM BETRIEB DER VORRICHTUNG**
DEVICE FOR CONTROLLED RELEASE OF SEVERAL ODORIFEROUS AND/OR ATTRACTIVE SUBSTANCES AND OPERATING METHOD
DISPOSITIF DE LIBERATION REGULEE DE PLUSIEURS SUBSTANCES ODORIFERANTES/ATTRACTIVES ET SON PROCEDE DE FONCTIONNEMENT

(30) Priorität: 18.09.2001 DE 10145954
(43) Veröffentlichungstag der Anmeldung: 23.06.2004
(73) Patentinhaber: Ruetz, Stefan, 80807 München (DE)
(72) Erfinder: Probst, Gerhard, 85716 Unterschleissheim (DE); Horn, Andreas, Dr., 85737 Ismaning (DE); Bock, Martin, 80804 München (DE); Kappel, Michael, Dr., 85386 Eching (DE)
(74) Vertreter: Zipse + Habersack
(86) Internationale Anmeldenummer: PCT/EP2002/010480
(87) Internationale Veröffentlichungsnummer: WO 2003/024493

(56) Entgegenhaltungen:
- EP-A- 0 627 224
- EP-A- 0 976 411
- US-A- 4 415 797
- US-A- 5 011 632
- US-A- 5 724 256

## Beschreibung

Die Erfindung bezieht sich auf eine Vorrichtung zum gesteuerten Freisetzen mehrerer Duft-/Lockstoffe durch Verdampfung, und auf ein Verfahren zum Betrieb der Vorrichtung.

Es sind mehrere Beduftungssysteme zur Raum- oder Personenbeduftung bekannt (z. B. aus EP-A 1 000 646), die Duftstoffe enthalten und diese als Dampf oder Aerosol freisetzen. Konstruktiv bedingt sind Raumbeduftungssysteme sehr träge, enthalten meist nur einen einzigen Duft und können keine Intensitätsprofile oder schnelle Duftwechsel realisieren. Sie scheiden damit für den reaktionsschnellen interaktiven Einsatz von Düften vollkommen aus, wie er beispielsweise als Ergänzung von Bild und Ton bei Film- oder Theatervorführungen, ja sogar am Fernseher zur Intensivierung des Miterlebens angestrebt wird. Für Insektenfallen sind andererseits Lockstoff-Ausbringvorrichtungen bekannt.

Anders als die Raumbeduftungssysteme sind Einzelpersonen- und Kleingruppen-Beduftungssysteme reaktionsschnell und bringen zielgerichtet zum Benutzer eine kleine Menge an Duftstoff aus. Einige dieser Systeme speichern und setzen auf Befehl auch viele verschiedene Duftstoffe frei (DE-A 199 38 405). Dies ermöglicht die erwähnte Untermalung von Ereignissen mit dazu passenden Düften z. B. für das Kino oder auch für Computerspiele. Die bisherigen Verfahren von Einzelpersonenbeduftungssystemen haben jedoch eine Reihen von Nachteilen. Diese liegen teilweise in der Vorratshaltung der Duftstoffe begründet. So werden Düfte in kleinen Portionen in "Chips" bevorratet und somit sind die Mengen zu gering, um mehrere Personen zu beduften oder über mehrere Tage ohne Wartung und Neubefüllung zu arbeiten. Bei diesem Verfahren haben die Duftstoffe auch beständig Kontakt zur Luft, d. h. die Duftstoffe verderben sehr schnell, verändern durch das vorzeitige Abdampfen leichtflüchtiger Duftstoffanteile ihren Dufteindruck und verursachen einen unangenehmen Dauergeruch bestehend aus allen gespeicherten Duftstoffen.

Besonders ausgeprägt ist dies bei Systemen (z.B. US-A-5724256), die ähnlich zu einigen Tintenstrahldruckern mit Piezospritzköpfen arbeiten. Diese benötigen immer eine offene Düse, um ausspritzen zu können, und eine offene Rückseite, um Luft nachziehen zu können, da sie nicht wie eine Pumpe eine Druckdifferenz aufbauen können. An diesen offenen Flächen dampfen die Duftstoffe ab, was zu den beschriebenen Nachteilen wie Dauergeruch und kurzer Lebensdauer der Duftstoffe führt. Durch die geringen Ausbringraten der Piezospritzköpfe muß zudem mit konzentrierten Duftstoffen gearbeitet werden, um eine akzeptable Duftintensität zu erzielen, was die Duftstoffe noch schneller verderben läßt, und eine Verwendung von Kunststoffen als Vorratstank aufwendig und die Verbindungstechnik zum Spritzkopf extrem schwierig macht, da viele konzentrierte Duftstoffe chemisch sehr aggressiv sind und fast alle Kunststoffe und Dichtmassen auflösen oder zum Quellen bringen.

Andere Duftausbringungssysteme zur Einzelpersonenbeduftung leiten Luft durch mit Duftstoff getränkte Poröskörper. Die Duftqualität ist bei diesen Systemen nicht konstant, da der Duftstoff fraktioniert ausdampft, d. h. die leichtflüchtigen Substanzen verdampfen schneller als die schwerflüchtigen, was eine ständige nachteilhafte Abweichung und Änderung des Dufteindruckes zur Folge hat. Die Ausbringung geringer Mengen Duftstoff für ausschließlich eine einzige Person ist damit nur mit hohem technischen Zusatzaufwand möglich. Außerdem sind diese Systeme aufwendig in der Installation und benötigen einen großen Bauraum.

Ein gemeinsames Problem der bis jetzt beschriebenen Duftgeräte ist die fehlende Möglichkeit zu überwachen, ob wirklich Duftstoff ausgebracht wird, und wenn ja, in welcher Menge. So kann es passieren, daß das Gerät Duft ausbringen soll, dies aber durch Luftblasen in den Zuleitungen oder durch einen defekten Ventilator gestört ist. Dies trifft vor allem für die Piezospritzköpfe zu, die bereits von kleinsten Luftblasen, die z. B. durch Erschütterungen oder beim Befüllen in das System gelangen können, außer Funktion gesetzt werden.

Ein weiteres gemeinsames Problem der bisherigen Duftsysteme ist die Anlagerung von Duftstoffrückständen im Inneren der Geräte, die Kontakt mit den verschiedenen bereits dampfförmigen Duftstoffen haben. Es kommt dadurch zu Nachgerüchen oder zu Mischgerüchen. Zudem sind sie prinzipbedingt teuer sowie schwierig und zeitaufwendig zu befüllen, und somit für den Massenmarkt ungeeignet.

Die hier beschriebene Erfindung beseitigt diese Probleme oder verringert sie deutlich, und weist zudem eine Reihe vollkommen neuer Funktionsmöglichkeiten auf. Die erfindungsgemäße Vorrichtung ist gekennzeichnet durch eine kontrolliert dosierende Fördereinrichtung umfassend eine mikrosystemtechnisch hergestellte Mikropumpe zur Förderung der flüssigen Duft- und Lockstoffe zu einem Verdampfer, und durch eine Einrichtung zur Erfassung der Verdampfungstemperatur unmittelbar am Verdampfer zur Betriebszustandsanalyse, und das erfindungsgemäße Verfahren zum Betrieb der Vorrichtung ist dadurch gekennzeichnet, daß man die Verdampfungstemperatur der Duftund/oder Lockstoffe mißt und unter Verwendung dieses Meßwerts die Verdampfungstemperatur und/oder die Pumprate und/oder die Verdampfungszeit regelt, und/oder daß man die Verdampfungstemperatur der Duft- und/oder Lockstoffe mißt und unter Verwendung dieses Meßwerts Fehler im Betriebsablauf identifiziert. Unter der "Verdampfungstemperatur" wird hierbei die Temperatur verstanden, die der Verdampfer hat oder die im unmittelbaren Verdampfungsbereich oder im diesen Bereich gerade verlassenden Dampf herrscht.

In der erfindungsgemäßen Vorrichtung werden also mikrosystemtechnische Mikropumpen für den Transport der flüssigen Duft- oder Lockstoffe eingesetzt. Diese verfügen im Gegensatz zum Piezospritzkopf über passive oder sogar aktive Ventile, die es ermöglichen, einen Saugdruck aufzubauen, und somit einen flexiblen, aber luftdichten Vorratsbehälter wie z. B. eine Falttube leerzusaugen. Die Flexibilität des Vorratsbehälters ist wichtig, da der Saugdruck der Mikropumpen vergleichsweise gering ist, und durch das Zusammenfalten des Vorratsbehälters dessen luftdichter Abschluß möglich ist. Durch im inaktiven Zustand geschlossene Ventile und die luftdichte Falttube ist der empfindliche Duft- oder Lockstoff hervorragend vor Umwelteinflüssen oder Eigenabdampfung geschützt, und die Umgebungsluft wird nicht durch ständiges Ausdampfen einer oder mehrerer Duftstoffe kontaminiert. Da Mikropumpen im Gegensatz zu Piezoköpfen viel höhere Flüssigkeitsmengen transportieren, können die Duftstoffe stark verdünnt werden, was deren chemische Aggressivität erheblich vermindert und den Einsatz von einfach auswechselbaren Kunststoffbehältern als Vorratsbehälter, also z. B. den Falttuben, erlaubt, da diese nun chemisch nicht mehr angegriffen werden. Zudem sind Mikropumpen relativ unempfindlich gegenüber Luftblasen, da diese durch die Ventile komprimiert und herausgepumpt werden können. Mikrosystemtechnische Mikropumpen sind dadurch gekennzeichnet, daß sie durch der Mikrosystemtechnik zugeordnete Fertigungsverfahren wie z. B. Strukturieren durch Masken und Ätzen, Mikrospritzgußtechnik, Mikroprägetechnik, Mikroabformtechnik oder auch LIGA-Technik hergestellt werden und aus Silicium, aber auch aus Kunststoffen wie z. B. Polypropylen bestehen. Herstellverfahren und Funktionsweise der Mikropumpen sind auch im Buch "Grundlagen der Mikrosystemtechnik", Hanser-Verlag München, auf Seiten 140 bis 142 dargestellt.

Das Zusammenspiel von Mikropumpen zusammen mit einer Temperaturerfassung direkt an den Verdampfern führt zu einer Reihe von wichtigen Vorteilen, da hierdurch alle Betriebszustände der Vorrichtung einfach kontrolliert und ausgeregelt werden können.

Die Tatsache, daß der verdampfte Duft- oder Lockstoff dem Verdampfer Wärme entzieht, ermöglicht es der Vorrichtung aufgrund der Erfassung der Verdampfertemperatur über einen Temperatursensor, Lufteinschlüsse in den Zuleitungen zu erkennen und durch Anheben der Pumprate, bis wieder Duft- oder Lockstoff den Verdampfer kühlt, selbsttätig zu beseitigen oder bei Mißerfolg eine größere Störung wie z. B. einen erschöpften Duftstoffvorrat zu detektieren, wenn über eine erhöhte Pumprate über längere Pumpzeit kein Duftstoff gefördert werden konnte, und über eine Schnittstelle nach außen zu melden. So kann im einfachen Fall der erschöpfte Duftstoffvorrat durch eine Leuchtanzeige kenntlich gemacht werden, oder es kann über einen Netzwerkanschluß die Servicezentrale des Betreibers über den zur Neige gehenden Duftstoffvorrat informiert werden. Ähnliches trifft auch zu, wenn z. B. der Ventilator ausfällt, oder die den gasförmigen Duft herauslassende Düse zum Benutzer z. B. von einem Kaugummi verschlossen ist. In beiden Fällen findet keine Kühlung des Heizfeldes bzw. des Verdampfers durch den vorbeistreichenden Luftstrom mehr statt, was über den Temperatursensor am Verdampfer sofort detektiert werden kann. Dies ermöglicht eine sofortige Information des Benutzers über der Fehlfunktion, und die betreffende Mikropumpe kann sofort abgeschaltet werden, damit das Innere des Gerätes nicht mit der nun nicht mehr heraustransportierten Duftwolke kontaminiert wird.

Gleiche Mikropumpen können fertigungsbedingt unterschiedliche Pumpraten haben. Die gleichbleibende Pumprate einer einzelnen Mikropumpe im Zusammenspiel mit der Erfassung der absoluten Pumprate über die Kühlwirkung erlaubt es, das bereits ausgebrachte Flüssigkeitsvolumen aufzusummieren und so eine Prognose über die noch verbliebene Flüssigkeitsmenge im Vorratsbehälter zu treffen. Dadurch kann eine kostengünstige Füllstandsanzeige realisiert werden.

Eine separate Beförderung der Duftstoffe vom Vorratsbehälter durch die Schläuche und durch separate Pumpen zu separaten Verdampfern verhindert die Entstehung von Mischgerüchen, da an keiner Stelle die flüssigen Duft- oder Lockstoffe vermischt werden. Um auch die evtl. im Luft-Strömungsrohr durch Ablagerung verschiedener Duftstoffe entstehenden Mischgerüche zu vermeiden, läßt sich auch das Strömungsrohr separat für jeden Duftstoff ausbilden und/oder aber auch die gemeinsame Düse über eine Flächenbeheizung oder einen warmen Luftstrom wie bei einem Warmluftgebläse verhindern.

Sollen Herstellungskosten gespart werden, so lassen sich mehrere Duftstoffe über ein Ventil von n auf m Leitungen, auch dieses bevorzugt in Mikrosystemtechnik hergestellt, durch weniger Mikropumpen als der Zahl der Duftstoffe oder auch nur durch eine einzige Mikropumpe befördern und in entsprechend weniger Verdampfereinheiten verdampfen. Eine weitere Kostenreduktion läßt sich durch weitgehende Integration der Einzelteile in hybrider oder monolithischer Bauweise erreichen, d. h. Integration der verwendeten Komponenten wie Mikropumpen und Verdampfer auf einem gemeinsamen Träger, oder mehreren Mikropumpen auf einem Träger.

Weitere Einzelheiten, Vorteile und Weiterbildungen der Erfindung ergeben sich aus der folgenden Beschreibung eines bevorzugten Ausführungsbeispiels unter Bezugnahme auf die Zeichnung. Es zeigen:
- Fig. 1: eine schematische Darstellung einer erfindungsgemäßen Vorrichtung zum Freisetzen von Duft- oder Lockstoffen;
- Fig. 2: ein Schema einer geräteaufwandsmäßig einfacheren Ausführung;
- Fig.n 3 bis 8: schematische Darstellungen von in der Vorrichtung von Fig. 1 verwendbaren Verdampfern;
- Fig.n 9 und 10: schematische Darstellungen von in der Vorrichtung von Fig. 1 verwendbaren Mikropumpen;
- Fig. 11: ein Ablaufdiagramm der von einer Steuerung nach Fig. 1 durchgeführten Verarbeitung.

Im folgenden wird anhand von Fig.n 1 und 2 die Funktionsweise des Vorrichtung beschrieben:

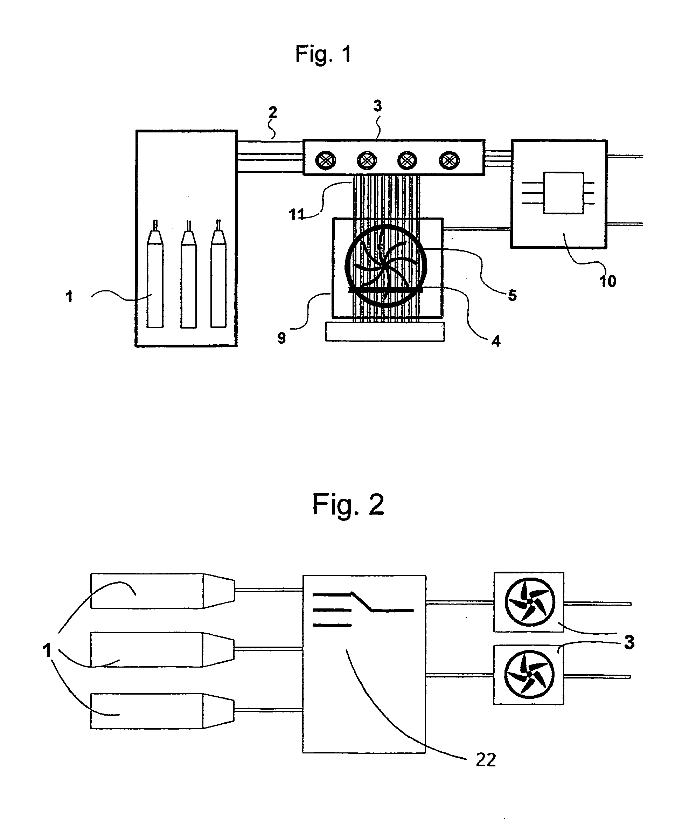

Aus Vorratsbehältern 1 wie Falttuben oder Tubenbeuteln oder ähnlichem fördern über Ansaugleitungen 2 mikrosystemtechnische Mikropumpen 3 flüssige Duft- oder Lockstoffe auf Verdampfer 4, die stromdurchflossene Metallröhrchen oder Heizfelder auf einem Träger, z. B. aus Keramik oder einem temperaturfesten Kunststoff, sein können. Ein Lüfter 5 fördert Umgebungsluft senkrecht zur Zeichnungsebene zu einer Duftauslaßdüse (nicht in der Zeichnung) über die Verdampfer 4, die sich in einem Strömungsrohr 9 befinden. In Fig. 1 nicht dargestellte Temperatursensoren messen laufend die Temperatur der einzelnen Verdampfer 4. Eine Steuerelektronik 10 übernimmt die Duftbefehle einer externen Anwendung und übernimmt die Meßwerte der Temperatur an den Verdampfern 4, und erzeugt die Pumpenspannung, bewertet die anhand der Temperatur ermittelten Betriebszustände und steuert entsprechend die Pumpen 3, die Lüfter 5 und die Heizfelder 4, und/oder gibt Zustandsmeldungen nach außen. Jede der Mikropumpen 3, jeder Verdampfer 4 und jeder Temperatursensor kann von der Steuerelektronik 10 einzeln angesteuert bzw. ausgelesen werden. Vorzugsweise hat jeder Duft- oder Lockstoff seine eigenen Komponenten, nämlich Mikropumpe, Leitungssystem und Verdampfer.

Fig. 2 zeigt ein Beispiel, bei dem durch ein n-auf-m-Ventil 22, das vorzugsweise in Mikrosystemtechnik hergestellt ist, zwei Pumpen 3 zur Ausbringung von drei verschiedenen Düften aus getrennten Falttuben 1 ausreichen. Dies hilft, auf Kosten der Duftqualität die Anzahl der Mikropumpen und damit die Fertigungskosten zu reduzieren.

Die Fig.n 3 bis 8 zeigen verschiedene Ausführungsbeispiele von Verdampfern. Die Fig.n 3 bis 8 haben gemeinsam, daß der jeweilige Verdampfer massearm und damit schnell ist, und sich in unmittelbarer Umgebung des Heizfeldes, an dem der flüssige Duft- oder Lockstoff verdampft, ein Temperatursensor befindet, bzw. die temperaturabhängige Widerstandsänderung des Heizwiderstandes selbst genutzt wird, um die Temperatur am Verdampfungspunkt zu erfassen.

Die Fig.n 3 bis 5 und 8 zeigen jeweils eine Zuleitung 7, die von der Mikropumpe kommt und flüssigen Duftstoff 8 auf das Heizfeld des Verdampfers 4 transportiert. In diesen Figuren ist der Verdampfer 4 aus Hybridkeramik 15 mit aufgerakeltem Heizfeld 4 und/oder Meßwiderständen 13 mit Anschlüssen 14, oder auch aus PTC-Keramik oder aus temperaturfester Folie oder Kunststoff mit aufgedampften oder galvanisch aufgebrachten und ätztechnisch strukturierten, metallischen Heizfeldern. Der Vorteil der Verdampfer nach den Fig.n 3 bis 5 und 8 ist, daß die Einzelverdampfer für die einzelnen Duft- oder Lockstoffe als Felder auf einer gemeinsamen Trägerplatte untergebracht werden können, was die Herstellung und Montage der Verdampfer vereinfacht und verbilligt und weniger Platz im Strömungsrohr 5 der Vorrichtung verbraucht.

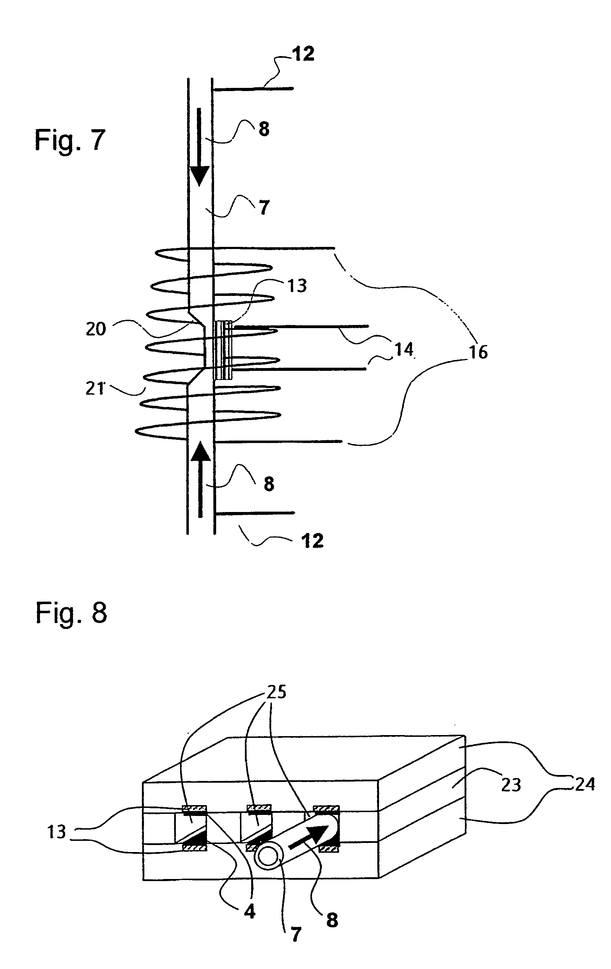

Fig. 8 zeigt ein Beispiel mit drei Verdampferkammern auf einem gemeinsamen Trägersubstrat aus z. B. Keramik. Die Fig.n 6 und 7 zeigen Verdampfer, bei denen die Zuleitung von der Pumpe, in Form eines metallischen Röhrchens 7, selbst als Verdampfer dient.

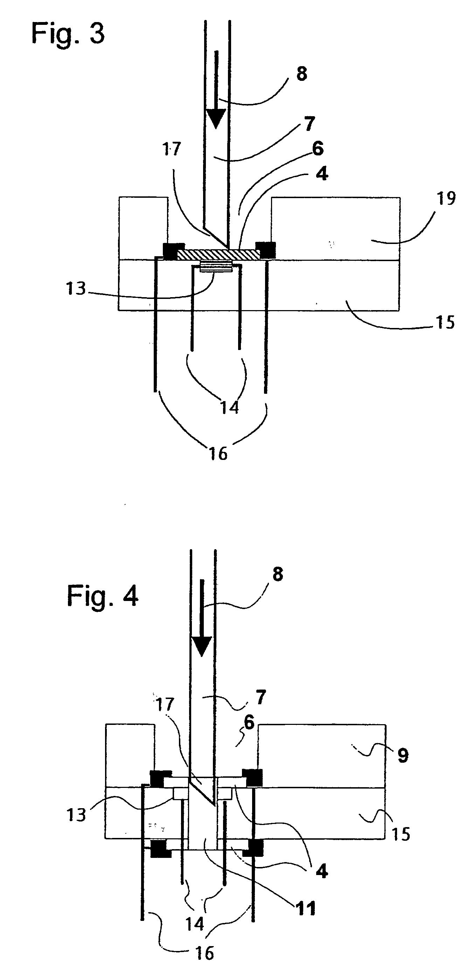

Im einzelnen besteht der Verdampfer nach Fig. 3 aus dem Verdampfer 4 in Form eines Heizfelds mit Anschlüssen 16 in einem Verdampfertopf 6 auf dem Träger 15 z. B. in Dickschichttechnik oder Folie. Der flüssige Duftstoff 8 wird durch das Röhrchen 7, das von der Pumpe 3 kommt und am unteren Ende 17 schräg abgeschnitten ist, gezielt in den Verdampfertopf 6 geleitet. Dort wird der Duftstoff auf dem Heizfeld erhitzt und somit verdampft. Über den Temperaturmeßwiderstand 13 und dessen Anschlüsse 14 liest die Steuerelektronik 10 die aktuelle Temperatur ein und speist entsprechend der Regellogik über die Anschlüssen 16 das Heizfeld. Eine Töpfchenplatte 19 verhindert das vorzeitige Abfließen des Duftstoffes.

Fig. 4 zeigt eine Verdampfereinheit ähnlich der in Fig. 3 mit dem Unterschied, daß der Duftstoff durch eine Bohrung 11 im Träger 15 hindurchfließt und diese Bohrung von beiden Seiten durch die Heizfelder 4 beheizt ist.

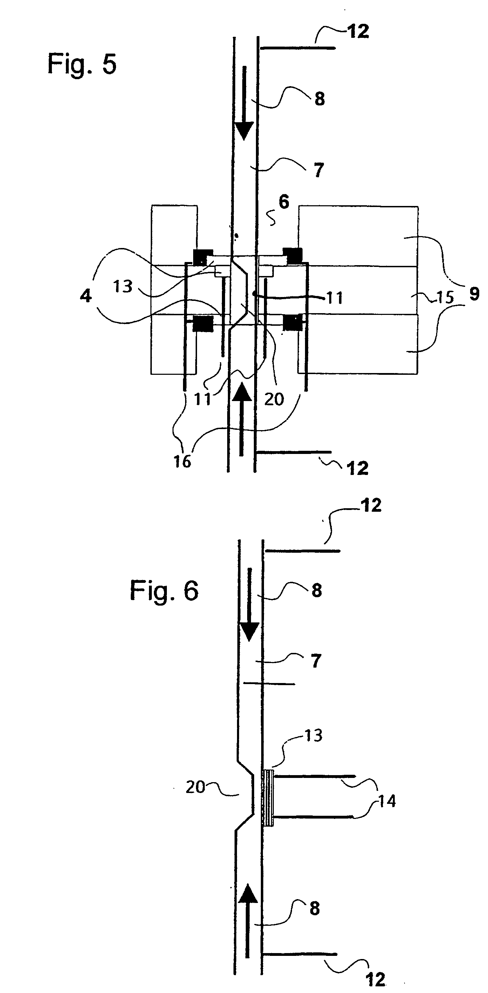

Fig. 5 zeigt ein Heizelement, das ähnlich wie in Fig. 4 aus dem gelochten Träger 15 besteht. Dabei können jedoch zwei verschiedene Duftstoffe 8 von beiden Seiten durch das in der Bohrung 11 bei 20 geöffnete Röhrchen 7 zum Heizfeld 4 fließen und dort verdampfen. Besteht das Röhrchen aus Metall, kann es zusätzlich über Anschlüsse 12 beheizt werden. Dies beschleunigt die Verdampfung und verhindert die Anlagerung von Duftstoff an das Röhrchen.

Fig. 6 zeigt wie Fig. 5 einen Verdampfer in Form des an einer Stelle 20 geöffneten Röhrchens 7, das durch elektrischen Strom über die Anschlüsse 12 beheizt wird. Der Duftstoff 8 fließt zu der durch die Öffnung 20 bewirkten Verengung, die die heißeste Stelle ist, und verdampft dort. Der Temperatursensor 13, der über die Anschlüsse 14 an die Steuerelektronik angeschlossen ist, erfaßt die Verdampfungstemperatur. Die Steuerelektronik regelt entsprechend der vorgegebenen Regellogik den Strom über die Anschlüsse 12.

Fig. 7 zeigt eine Verdampfereinheit wie in Fig. 6, die über eine zusätzliche Heizwicklung 21 das Röhrchen auch von außen erhitzt.

Fig. 8 zeigt einen Verdampfer aus mehreren Schichten 23, 24 aus Hybridkeramik oder Folie. Auf der oberen und unteren Schicht 24 sind die Heiz- und Meßwiderstände untergebracht. Die Zwischenschicht 23 ist kammartig und erzeugt Kavitäten 25, die von den Heizwiderständen des Verdampfers 4 einzeln beheizt werden können. Die Röhrchen 7 sind Kapillare, die von der Seite in den Mehrfachverdampferblock eingeführt werden können, wo sie den flüssigen Duftstoff 8 in die Kavität 25 abgeben. Dieser Aufbau ist sehr montagefreundlich beim Einsetzen der Kapillare, da es keine Rolle spielt, ob die Kapillare z. B. ein oder drei mm in den Block hineinsteht. Sind die Kavitäten nicht durchgehend, sondern wie ein Sackloch angelegt, dann ist es sehr unwahrscheinlich, das noch unverdampfte Duftstofftröpfchen aus der Kavität 11 herausspritzen und die Umgebung kontaminieren. Die Heiz- und Meßwiderstände jeder einzelnen Kavität sind durch getrennte Leitungen einzeln ansteuerbar, bzw. auslesbar.

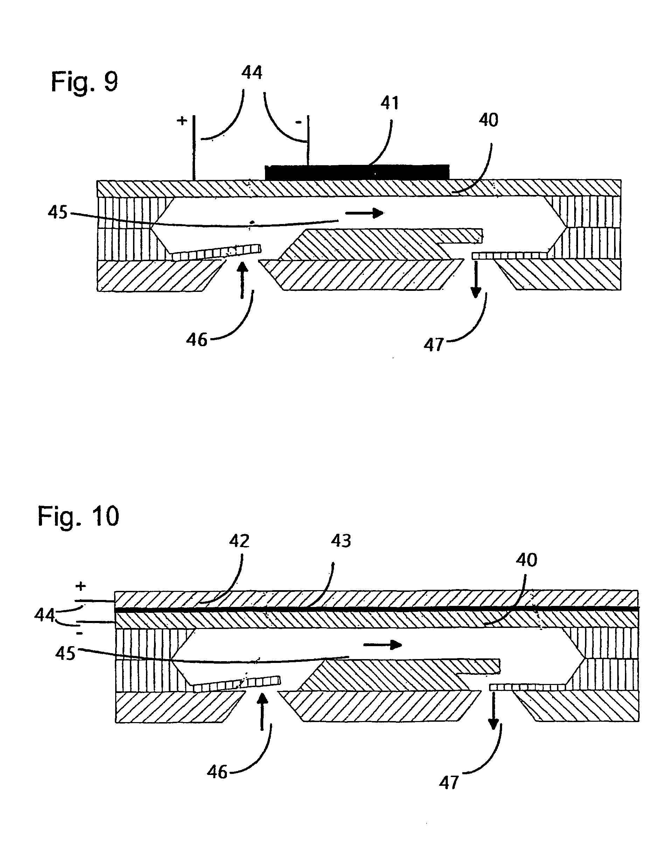

Die Fig. 9 zeigt beispielhaft den Prinzipaufbau der verwendeten Pumpen mit Piezoantrieb, und Fig. 10 diesen mit einem elektrostatischem Antrieb. Das Pumpen geschieht durch eine Membran 40, die verformt wird durch einen Piezo 41 bzw. auch durch eine Gegenelektrode 42 über einem Isolator 43, die über Spannungskontakte 44 mit einer Spannung beaufschlagt werden und ein Zusammenkrümmen der Membran 40 verursachen. Wenn sich die Membran 40 nach oben wölbt, wird in eine Pumpkammer 45 über ein Einlaßventil 46 flüssiger Duft- oder Lockstoff eingesaugt. Beim Anlegen einer entgegengerichteten Spannung an den Piezo 41 oder an die Gegenelektrode 42 schließt sich das Einlaßventil 46 durch den Druck, und ein Auslaßventil 47 öffnet sich. Durch die Abwärtsbewegung der Membran wird der Duft- oder Lockstoff aus der Pumpkammer 45 über das Auslaßventil 47 herausgedrückt, der Pumpzyklus kann nun von neuem beginnen.

Bei den Fig.n 9 und 10 sind Mikropumpen mit passiven Ventilen dargestellt, die allein durch Saug- oder Preßdruck geöffnet oder geschlossen werden. Es gibt jedoch auch Mikropumpen mit aktiv angesteuerten Ventilen, die je über einen eigenen Aktor (meist Piezoaktor) geöffnet und geschlossen werden können. Diese Mikropumpen verfügen aktiv geschlossen über eine niedrigere Leckrate und können außerdem vorwärts und rückwärts pumpen.

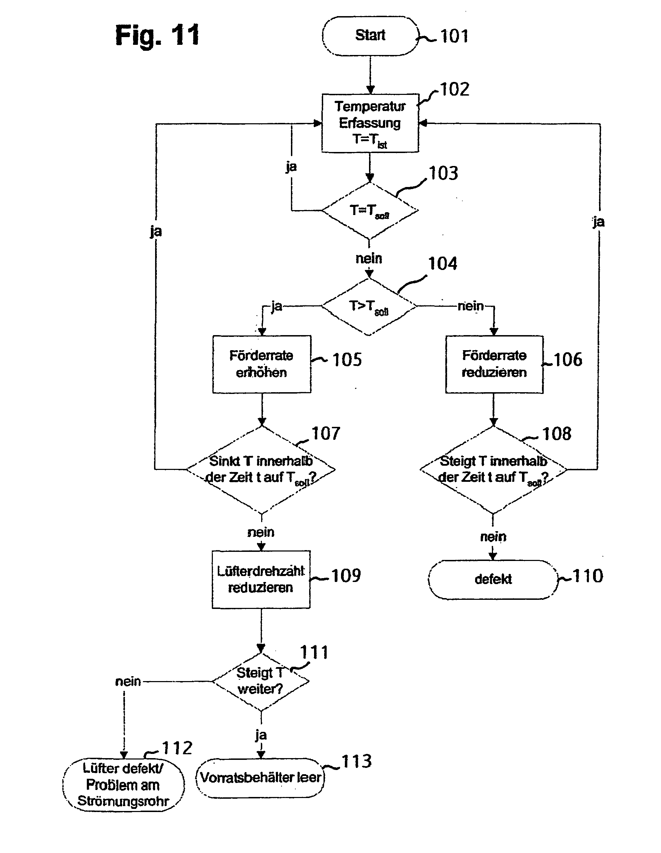

Fig. 11 zeigt ein Diagramm, das die Verfahren der Fehlererkennung erläutert.

Ausgangspunkt der Fehlererkennung ist generell die Temperaturerfassung 102 über den Temperatursensor 13 am Heizfeld, bzw. an der geöffneten Stelle 20 des metallischen Röhrchens 7.

Die Temperatur wird erfaßt und mit der Solltemperatur verglichen (Schritt 103). Stimmt die erfaßte Temperatur mit der Solltemperatur überein, so ist alles in Ordnung. Die Temperaturerfassung wird in regelmäßigen Abständen wiederholt.

Ist die erfaßte Temperatur höher als die Solltemperatur, wird zunächst die Förderrate der Pumpen erhöht (Schritt 105). Eventuell vorhandene Luftblasen, wie sie z. B. direkt nach dem Einschalten des Gerätes, nach Erschütterungen oder nach dem Wechsel des Vorratsbehälters vorhanden sein können, werden dadurch schnell ausgeblasen.

Innerhalb einer bestimmten vorher festgelegten Zeit muß die Temperatur auf den Sollwert absinken bzw. die Regelgröße Heizleistung ansteigen - Flüssigkeit wird wieder verdampft, der Fehler ist behoben (Schritt 107). Diese Zeit hängt z. B. von der Länge der Zuleitungen und deren Querschnitt ab. Sinkt die erfaßte Temperatur innerhalb dieser Zeit nicht ab oder steigt die Regelgröße Heizleistung nicht an, wird die Drehzahl des Lüfters, der den Transportstrom durch das Luft-Strömungsrohr erzeugt, reduziert (Schritt 109). Sinkt die Temperatur trotzdem nicht (Schritt 112), so liegt ein Defekt am Lüfter oder eine Störung am Strömungsrohr vor, z. B. eine durch einen Finger oder Kaugummi verstopfte Austrittsöffnung des Strömungsrohrs. Sinkt die Temperatur dadurch nochmals, so ist nur der Vorratsbehälter für die Flüssigkeit leer (Schritt 113) und muß getauscht oder aufgefüllt werden.

Ist die erfaßte Temperatur niedriger als die Solltemperatur, wird die Förderrate der Pumpe reduziert (Schritt 106). Im Übermaß ausgetretene Flüssigkeit kann dadurch schneller verdampfen. Nach einer vorher festgelegten Zeit sollte die erfaßte Temperatur auf oder sogar über den Sollwert ansteigen (Schritt 108). Die im Übermaß ausgetretene Flüssigkeit ist verdampft, die Pumpe wird auf die ursprüngliche Förderrate zurückgestellt. Steigt die erfaßte Temperatur innerhalb dieser Zeit nicht an, liegt ein anderer Fehler vor (Schritt 110).

Durch die Erfindung ergeben sich folgende Vorteile im Vergleich zu bestehenden Technologien:
- Lange Haltbarkeit und Lebensdauer des Duft- bzw. Lockstoffvorrates, da die Falttuben in fast beliebigen Größen existieren, und als lebensmittelgeeignete Behälter weder den Inhalt chemisch beeinflussen noch Luft- und Lichtzutritt erlauben. Dadurch wird nicht nur die gleichbleibende Qualität der Duft- bzw. Lockstoffe ermöglicht, sondern auch die Eigenabdampfung und der damit einhergehende Mischgeruch verhindert.
- Füllstands- und Statusanzeigen
- Ausgleich fertigungsbedingter Schwankungen des Dufttransportmechanismus (hier Mikropumpe) und dessen möglicher Degradation über die Lebensdauer durch Alterung oder durch Umwelteinflüsse (Temperatur, Luftfeuchtigkeit usw.)
- Es kann exakt bestimmt werden, ob und wie viel Duft die Vorrichtung tatsächlich ausgibt, Betriebszustände können ausgegeben und Fehlerzustände beseitigt werden. So kann sich die Vorrichtung z. B. beim Einschalten oder nach dem Befüllen automatisch initialisieren, d. h. so lange pumpen, bis Duftstoff oder Lockstoff an dem jeweiligen Verdampfer ansteht.
- Die Vorrichtung kann wenige bis viele Duftstoffe auf kleinem Raum in vollkommen getrennter Weise beherbergen und diese in sekundenschneller Wechselfolge ausbringen.
- Die Vorrichtung kann von ungeschultem Personal nachgefüllt werden, da Falttuben aus dem Alltag bekannt und leicht zu handhabende Vorratsbehälter sind.
- Die Vorrichtung taugt durch ihren Funktionsumfang für einen breiten Anwendungsbereich und kann mit unverändertem Grundprinzip als Tischgerät oder als Einbaugerät für Spielautomaten, Anzeigeeinheiten, PCs, Spielekonsolen, Terminals, Autos, Fitneßgeräte, Wellneßtherapiegeräte usw. ausgeführt werden.
- Durch seine hohe Dynamik in der Menge des ausbringbaren Duftstoffes kann die Vorrichtung zur reaktionsschnellen Einzel- oder Kleingruppenbeduftung, oder aber auch als variabler Raumbedufter eingesetzt werden.

## Patentansprüche

1. Vorrichtung zum gesteuerten Freisetzen mehrerer Duft- und/oder Lockstoffe durch Verdampfung, **gekennzeichnet durch** eine kontrolliert dosierende Fördereinrichtung umfassend einen Verdampfer (4) und eine mikrosystemtechnisch hergestellte Mikropumpe (3) mit passiven oder aktiven Ventilen (22) zur Förderung der flüssigen Duft- und Lockstoffe zum Verdampfer (4), und **durch** eine Einrichtung (13) zur Erfassung der Verdampfungstemperatur unmittelbar am Verdampfer zur Betriebszustandsanalyse.

2. Vorrichtung nach Anspruch 1, **gekennzeichnet durch** ein oder mehrere einzeln ansteuerbare ebene Heizfelder (4) in Dickschicht-Keramik- oder PTC-Keramik- oder Heizfolien-Ausführung zur Erhitzung der flüssigen Duft- und/oder Lockstoffe, und **durch** in der unmittelbaren Nachbarschaft jedes Heizfeldes angeordnete temperaturempfindliche Elemente (13).

3. Vorrichtung nach Anspruch 1, **gekennzeichnet durch** ein oder mehrere einzeln ansteuerbare ebene Heizfelder (4) in Dickschicht-Keramik- oder PTC-Keramik- oder Heizfolien-Ausführung zur Erhitzung der flüssigen Duft- und/oder Lockstoffe, und **durch** eine Meßeinrichtung zur Messung der temperaturabhängigen Widerstandesänderung des Heizfeldes.

4. Vorrichtung nach einem der Ansprüche 1 bis 3, **dadurch gekennzeichnet, daß** mehrere Verdampfer vorhanden sind und die Verdampfer jeweils ein metallisches Röhrchen (7) sind, durch das die Stoffe (8) zu pumpen sind, das von elektrischem Strom durchflossen und dadurch erhitzt ist und an dem an einer halbseitigen Öffnung (20) ein Ort erhöhter Temperatur entsteht, an dem der flüssige Lock- und/oder Duftstoff verdampft und in Luft übergeht.

5. Vorrichtung nach einem der Ansprüche 1 bis 4, **dadurch gekennzeichnet, daß** Heizvorrichtungen vorhanden sind, die durch Erwärmung von im Luftströmungskanal (9) befindlichen Bauteilen, durch Erwärmung des Luftstromes und/oder durch Oberflächenbeheizungen die Ablagerung von Duftstoff- oder Lockstoffrückständen auf den Oberflächen innerhalb des Strömungskanals minimieren.

6. Vorrichtung nach einem der Ansprüche 1 bis 5, **dadurch gekennzeichnet, daß** mehrere Vorratsbehälter (1), mehrere Mikropumpen (3) und mehrere Verdampfereinheiten (4) für die Lock- und/oder Duftstoffe vorhanden und durch eine Steuereinheit so gesteuert sind, daß die verschiedenen Lockund/oder Duftstoffe zeitlich versetzt verdampft werden.

7. Vorrichtung nach Anspruch 6, **gekennzeichnet durch** Verwendung eines n-auf-m-Umschaltventils (22), **durch** das n Flüssigkeiten, befördert von m Mikropumpen (3), auf m Verdampfereinheiten (4) ausbringbar sind.

8. Vorrichtung nach Anspruch 6 oder 7, **dadurch gekennzeichnet, daß** die Pumpen (3), Ventile (22) und Förderleitungen (7), hybrid oder monolithisch, in Mikrosystemtechnik auf einem gemeinsamen Träger ausgeführt sind.

9. Vorrichtung nach einem der Ansprüche 1 bis 8, **dadurch gekennzeichnet, daß** mit der Einrichtung/den Einrichtungen (13) zur Erfassung der Verdampfungstemperatur eine Betriebsanalyse- und Fehleranalyseeinheit (10) verbunden ist, die wiederum mit Betriebszustand- und Fehleranzeigevorrichtungen verbunden ist.

10. Vorrichtung nach einem der Ansprüche 1 bis 9, **dadurch gekennzeichnet, daß** Vorratsbehälter (1) für die flüssigen Duftstoffe und/oder Lockstoffe (8) Falttuben oder Faltbeutel sind.

11. Vorrichtung nach Anspruch 10, **dadurch gekennzeichnet, daß** jede Falttube bzw. jeder Faltbeutel und die dazugehörige Mikropumpe eine gemeinsame Einheit sind, die vorzugsweise zusammengesteckt oder miteinander verschraubt sind.

12. Vorrichtung nach einem der Ansprüche 1 bis 11, **dadurch gekennzeichnet, daß** die Vorrichtung zu ihrer Energieversorgung mit einer mobilen Energiequelle verbunden ist.

13. Vorrichtung nach einem der Ansprüche 1 bis 12, **dadurch gekennzeichnet, daß** mehrere Verdampfer (4) vorhanden sind und die Verdampfer (4) Heizfelder sind, die eine so geringe Masse aufweisen, daß sie in weniger als drei Sekunden und mit weniger als 5 Watt Heizleistung eine Temperatur von über 100° C erreichen.

14. Verfahren zum Betrieb der Vorrichtung nach einem der Ansprüche 1 bis 13, **dadurch gekennzeichnet, daß** man die Verdampfungstemperatur der Duft- und/oder Lockstoffe mißt und unter Verwendung dieses Meßwerts die Verdampfungstemperatur und/oder die Pumprate und/oder die Verdampfungszeit regelt.

15. Verfahren insbesondere nach Anspruch 14, zum Betrieb der Vorrichtung nach einem der Ansprüche 1 bis 13, **dadurch gekennzeichnet, daß** man die Verdampfungstemperatur der Duft- und/oder Lockstoffe mißt und unter Verwendung dieses Meßwerts Fehler im Betriebsablauf identifiziert.

16. Verfahren nach Anspruch 15, **dadurch gekennzeichnet, daß** die Fehler, deren Vorliegen man durch einen Temperaturanstieg erkennt, austretende Lufteinschlüsse aus der Fördereinrichtung umfassen, die man durch Erhöhung der Förderrate schnell ausbläst.

17. Verfahren nach Anspruch 15 oder 16, **dadurch gekennzeichnet, daß** die Fehler, deren Vorliegen man durch eine Temperaturänderung erkennt, Störungen eines Ventilators oder eine Verstopfung der Luftaustrittsöffnung des Luftströmungskanals umfassen.

18. Verfahren nach einem der Ansprüche 15 bis 17, **dadurch gekennzeichnet, daß** die Fehler, deren Vorliegen man durch eine Temperaturänderung erkennt, eine Erschöpfung des Duftstoff- und/oder Lockstoffvorrates und/oder noch vorhandene Flüssigkeitsreste am Verdampfer umfassen.

19. Verfahren nach Anspruch 14 oder nach einem der auf Anspruch 14 rückbezogenen Ansprüche 15 bis 18, **dadurch gekennzeichnet, daß** man anhand der Temperatur-Meßwerte den Durchsatz an flüssigem Duft- und Lockstoff erkennt und die Pumprate der Mikropumpen regelt, wobei man den Durchsatz-Sollwert zum Ausgleich fertigungsbedingter Streuungen der Pumprate der Mikropumpen und/oder personenbezogener Empfindlichkeitsunterschiede des Geruchsempfindens einstellt.

20. Verfahren nach einem der Ansprüche 14 bis 19, **dadurch gekennzeichnet, daß** man durch einen beheizten Luftstrom durch die Vorrichtung oder durch Flächenbeheizung ein Niederschlagen von Duftstoffdampf im Inneren der Vorrichtung verhindert.

## Claims

1. A device for controlled release of several fragrances and/or pheromones by evaporation, **characterized by** a controlled dispensing means comprising an evaporator (4) and a micropump (3) in microsystem technology including passive or active valves for dispensing the liquid fragrances and pheromones to the evaporator (4), and by a means (13) for sensing the evaporation temperature directly at the evaporator for analysis of the operating condition.

2. The device as set forth in claim 1, **characterized by** one or more individually controllable thick-layer, ceramic or PTC ceramic or foil-type planar heaters for heating said liquid fragrances and/or pheromones, and by temperature-sensitive elements (13) arranged in the direct vicinity of each heater.

3. The device as set forth in claim 1, **characterized by** one or more individually controllable thick-layer, ceramic or PTC ceramic or foil-type planar heaters for heating said liquid fragrances and/or pheromones, and by a means for measuring the change in resistance of said heater as a function of the temperature.

4. The device as set forth in any of the claims 1 to 3, **characterized in that** several evaporators are provided, each of which is a metallic tube (7) through which said substances (8) are to be pumped with a flow of electric current therethrough in heating thereof and at a semi-side aperture (20) a high temperature location materializes at which said liquid fragrance and/or pheromone evaporates in translating into the air.

5. The device as set forth in any of the claims 1 to 4, **characterized in that** heater means are provided, which by heating components in the air flow conduit (9), by heating said air flow and/or by surface heating minimize deposits of fragrance and/or pheromone remainders on the surfaces within the flow conduit.

6. The device as set forth in any of the claims 1 to 5, **characterized in that** several reservoirs (1), several micropumps (3) and several evaporator units (4) are provided for said fragrances and/or pheromones and are controlled by a controller so that the various fragrances and/or pheromones are evaporated staggered in time.

7. The device as set forth in claim 6, **characterized by** use of an n-to-m selector valve (22) through which n liquids dispensed by m micropumps (3) are deliverable to m evaporator units (4).

8. The device as set forth in claim 6 or 7, **characterized in that** said pumps (3), valves (22) and conduits (7) are configured hybrid or monolithic in microsystem technology on a common substrate.

9. The device as set forth in any of the claims 1 to 8, **characterized in that** connected to said means (13) for sensing said evaporation temperature is an operation/error analyzer (10) which is in turn connected to operation/error indicators.

10. The device as set forth in any of the claims 1 to 9, **characterized in that** said reservoirs (1) for said liquid fragrances and/or pheromones (8) are foldable tubes or packs.

11. The device as set forth in claim 10, **characterized in that** each foldable tube or foldable pack and its corresponding micropump forms a common unit preferably plugged or screwed together.

12. The device as set forth in any of the claims 1 to 11, **characterized in that** for its energy supply said device is connected to a mobile source of energy.

13. The device as set forth in any of the claims 1 to 12, **characterized in that** several evaporators (4) are provided and said evaporators (4) are heaters of such low mass that they attain a temperature exceeding 100°C in less than 3 seconds with a heating power of less than 5 watt.

14. A method for operating the device as set forth in any of the claims 1 to 13, **characterized in that** said evaporation temperature of said fragrances and/or pheromones is measured and the result used to regulate said evaporation temperature and/or pumping rate and/or evaporation time.

15. The method as set forth in claim 14 for operating the device as set forth in any of the claims 1 to 13, **characterized in that**, said evaporation temperature of said fragrances and/or pheromones is measured and the result used to identify errors in the operating sequence.

16. The method as set forth in claim 15, **characterized in that** said errors as detected by an increase in temperature, involve air inclusions from the dispensing means which are quickly blown out by increasing the dispensing rate.

17. The method as set forth in claim 15 or claim 16, **characterized in that** said errors as detected by an increase in temperature, involve a fan fault or blockage of the air outlet of said air flow conduit.

18. The method as set forth in any of the claims 15 to 17, **characterized in that** said errors as detected by an increase in temperature, involve depletion of said fragrance and/or pheromone supply and/or liquid remainders still existing in the evaporator.

19. The method as set forth in claim 14 or as set forth in any of the claims 15 to 18 relating back to claim 14, **characterized in that** the results of measuring the temperature are used to detect the flow of liquid fragrance and/or pheromone and to regulate said pumping rate, the ideal flow being set to compensate dispersions in the pumping rate of said micropumps stemming from manufacturing tolerances and/or differences in personal olfactory sensitivity.

20. The method as set forth in any of the claims 14 to 19, **characterized in that** precipitation of fragrance vapor in the interior of the device is prevented by passing a heated flow of air through the device or by surface heating.

## Revendications

1. Dispositif pour la libération contrôlée de plusieurs parfums et/ou attractifs par évaporation, **caractérisé par** un dispositif d'alimentation à dosage contrôlé comprenant un évaporateur (4) et une micro-pompe fabriquée selon la microtechnique avec des soupapes passives ou actives pour transporter les parfums et les attractifs liquides vers l'évaporateur (4), et comprenant un dispositif (13) pour la détermination de la température de l'évaporation directement sur l'évaporateur afin d'analyser l'état de fonctionnement.

2. Dispositif selon la revendication 1, **caractérisé par** un ou plusieurs zones de chauffage (4) planes susceptibles d'être commandés séparément dans une réalisation en céramique à couche épaisse ou en céramique PTC ou en feuilles chauffantes pour chauffer les parfums et/ou les attractifs, et **caractérisé par** des éléments (13) sensibles à la température disposés dans le voisinage direct de chaque zone de chauffage.

3. Dispositif selon la revendication 3, **caractérisé par** un ou plusieurs zones de chauffage (4) planes susceptibles d'être commandés séparément dans une réalisation en céramique à couche épaisse ou en céramique PTC ou en feuilles chauffantes pour chauffer les parfums et/ou attractifs, et **caractérisé par** un dispositif de mesure pour mesurer le changement de résistance lié à la température due la zone de chauffage.

4. Dispositif selon une des revendications 1 à 3, **caractérisé en ce qu'**il existe plusieurs évaporateurs et qu'ils sont constitués d'un petit tube (7) en métal, au travers duquel les matières passent pour aller vers la pompe, lequel petit tube est traversé par un courant électrique qui le chauffe et sur lequel une zone de température élevée se forme sur une ouverture (20) sur un demi côté où l'attractif et / ou le parfum liquide s'évapore et se dissipe dans l'air.

5. Dispositif selon une des revendications 1 à 4, **caractérisé en ce qu'**il existe des dispositifs de chauffage qui minimisent le dépôt de résidus de parfum ou d'attractif sur les surfaces à l'intérieur du canal en chauffant des éléments présents dans le canal de circulation de l'air (9), en chauffant le courant d'air et/ou en chauffant les surfaces.

6. Dispositif selon une des revendications 1 à 5, **caractérisé en ce qu'**il existe plusieurs réservoirs (1), plusieurs micro-pompes (3) et plusieurs unités d'évaporation (4) pour les attractifs et/ou les parfums et que ceux-ci sont commandés par une unité de commande de manière à ce que les attractifs et/ou les parfums se trouvent évaporées en étant décalées dans le temps.

7. Dispositif selon la revendication 6, **caractérisé par** l'utilisation d'une soupape d'inversion n à m (22) à travers laquelle il est possible d'introduire n liquides, transportés par n micro-pompes (3) sur m unités d'évaporation (4).

8. Dispositif selon la revendication 6 ou 7, **caractérisé en ce que** les pompes (3), les soupapes (22) et les conduites d'alimentation (7) sont réalisées de façon hybride ou monolithique selon une microtechnique sur un support commun.

9. Dispositif selon une des revendications 1 à 8, **caractérisé en ce qu'**une unité d'analyse du fonctionnement et d'analyse d'erreur (10) est reliée au dispositif/aux dispositifs (13) afin de déterminer la température d'évaporation, laquelle unité est à son tour reliée à des dispositifs vérifiant l'état de fonctionnement et indiquant les erreurs.

10. Dispositif selon une des revendications 1 à 9, **caractérisé en ce que** les réservoirs (1) pour les parfums et/ou les attractifs (8) sont des tubes à pliage ou des sacs à pliage.

11. Dispositif selon la revendication 10, **caractérisé en ce que** le chaque tube à pliage , respectivement chaque sac à pliage et la pompe associée forment une unité commune, qui sont, de préférence, enfichés ou vissés ensemble.

12. Dispositif selon une des revendications 1 à 11, **caractérisé en ce que** le dispositif est relié pour son alimentation en énergie avec une source d'énergie mobile.

13. Dispositif selon une des revendications 1 à 12, **caractérisé en ce qu'**il existe plusieurs évaporateurs (4) et **en ce que** les évaporateurs sont des zones de chauffage présentant une masse si petite capable d'atteindre une température supérieure à 100°C en moins de trois secondes et avec moins de 5 W de puissance de chauffage.

14. Procédé pour le fonctionnement du dispositif selon une des revendications 1 à 13, **caractérisé en ce qu'**on mesure la température d'évaporation des parfums et/ou des attractifs et **en ce qu'**on règle la température d'évaporation et/ou le débit de pompe et/ou la durée d'évaporation en utilisant cette valeur de mesure.

15. Procédé, notamment selon la revendication 14, pour le fonctionnement du dispositif selon une des revendications 1 à 13, **caractérisé en ce qu'**on mesure la température d'évaporation des parfums et/ou des attractifs et **en ce qu'**on identifie des erreurs dans le déroulement du fonctionnement en utilisant cette valeur de mesure.

16. Procédé selon la revendication 15, **caractérisé en ce que** les erreurs dont on reconnaît l'existence par une augmentation de la température, incluent des connexions d'air sortant du dispositif d'alimentation que l'on vide en augmentant les cadences d'alimentation.

17. Procédé selon la revendication 15 ou 16, **caractérisé en ce que** les erreurs que l'on reconnaît par la présence d'un changement de température, comprennent des perturbations provenant d'un ventilateur ou un encombrement de l'ouverture de sortie de l'air du canal de circulation d'air.

18. Procédé selon une des revendications 15 à 17, **caractérisé en ce que** les erreurs que l'on reconnaît par un changement de température, incluent un tarissement de la réserve de parfums et/ou d'attractifs et/ou des restes de liquide encore présents dans l'évaporateur.

19. Procédé selon la revendication 14 ou selon une des revendications 15 à 18 se référant à la revendication 14, **caractérisé en ce que** l'on reconnaît le débit de parfums et d'attractifs liquides à l'aide de valeurs de température et **en ce qu'**on règle le débit de pompe des micro-pompes ; la valeur de consigne du débit destinée à équilibrer des variations, liées à la fabrication, du débit de pompe des micro-pompes et/ou des différences de sensibilité de perception de l'odeur liées aux personnes étant réglée.

20. Procédé selon une des revendications 14 à 19, **caractérisé en ce qu'**on empêche une accumulation de la vapeur du parfum à l'intérieur du dispositif grâce à un courant d'air chauffé par le dispositif ou grâce à un chauffage de la surface.
